(19) Europäisches Patentamt
European Patent Office
Office européen des brevets

(11) **EP 2 682 470 A1**

(12) **EUROPÄISCHE PATENTANMELDUNG**

(43) Veröffentlichungstag:
**08.01.2014 Patentblatt 2014/02**

(51) Int Cl.:
***C12P 5/02*** *(2006.01)*

(21) Anmeldenummer: **12005002.6**

(22) Anmeldetag: **05.07.2012**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **ISF GmbH**
**25421 Pinneberg (DE)**

(72) Erfinder:
• **Mathies, Edmund**
**25436 Moorrege (DE)**

• **Ramhold, Dietmar**
**23820 Pronstorf (DE)**
• **Winkelmann, Jörg**
**04451 Borsdorf (DE)**

(74) Vertreter: **Siemons, Norbert**
**Hauck Patent- und Rechtsanwälte**
**Neuer Wall 50**
**20354 Hamburg (DE)**

(54) **Verfahren zur Erzeugung von Biogas aus Verarbeitungsrückständen von Früchten oder Rüben oder Knollen oder aus unverarbeiteten Früchten oder Rüben oder Knollen als zu vergärendes Ausgangssubstrat in einem Biogasreaktor**

(57) Verfahren zur Erzeugung von Biogas aus Verarbeitungsrückständen von Früchten oder Rüben oder Knollen oder aus unverarbeiteten Früchten oder Rüben oder Knollen als zu vergärendes Ausgangssubstrat in einem Biogasreaktor, bei dem im Falle einer Unterschreitung eines Mindestwertes der Ammonium-Stickstoff-Konzentration im Gärsubstrat ein Prozesshilfsstoff zugeführt wird, aus dem keine oder keine nennenswerten Biogasmengen gebildet werden und der einen im Gärsubstrat durch Desaminierung Ammonium-Stickstoff freigebenden Stoff umfasst.

Fig. 1

EP 2 682 470 A1

## Beschreibung

Die Erfindung betrifft ein Verfahren zur Erzeugung von Biogas aus Verarbeitungsrückständen von Früchten oder Rüben oder Knollen oder aus unverarbeiteten Früchten oder Rüben oder Knollen als zu vergärendes Ausgangssubstrat in einem Biogasreaktor.

Die Fixierung von Sonnenenergie in Biomasse durch die pflanzliche Photosynthese ist eine der bedeutendsten, sich selbst erneuernden Energiequellen (Maurer, M. und Winkler, J.-B.; Biogas. Theoretische Grundlagen, Bau und Betrieb von Anlagen; 1982; Springer-Verlag). Aufbauend auf der Energiegewinnung durch die Photosynthese werden als Stoffwechselleistung von den Pflanzen Makromoleküle synthetisiert. Diese Makromoleküle können beim anaeroben Abbau in Biogasanlagen mit einer sehr hohen Effizienz in Methan und Kohlendioxid umgewandelt werden, sodass bis zu 82% der in den Pflanzen gespeicherten Energie in Methan überführt werden. Der Prozess der Biogaserzeugung kann in vier Stufen unterteilt werden. In einem ersten Schritt, der Hydrolyse, werden die komplexen Strukturen der Biomasse in ihre Monomere (Zucker, Fette, Eiweiße) zerlegt. Anschließend erfolgt ein Abbau der Monomere zu kurzkettigen Fettsäuren (Acidogenese). Im dritten (Acetogenese) und vierten Schritt (Methanogenese) erfolgt zunächst die Bildung von Essigsäure und in der Folge von Methan. Als Nebenprodukte entstehen im Biogasprozess vor allem Kohlendioxid und weitere Gase in geringen Konzentrationen. Die optimalen Milieubedingungen unterscheiden sich in den jeweiligen Schritten zum Teil erheblich (Sahm: Biologie der Methanbildung, Chem.-Ing. Tec. 53 (1981) Nr. 11, S. 854-863).

Gemäß dem Stand der Technik erfolgt der anaerobe Abbau organischer Substanz im wässrigen Milieu mit Trockensubstanzgehalten von in der Regel unter 30%.

Die Erzeugung von Biogas erfolgt in Abhängigkeit von den am Prozess beteiligen Mikroorganismen bei verschiedenen Temperaturoptima im Bereich von 25° bis 57°C.

Das optimale Kohlenstoff:Stickstoff:Phosphor:Schwefel-Verhältnis liegt bei 500:15:5:3 für die Hydrolyse und Acidogenese, bzw. 600:15:5:3 für die Acetogenese und Methanogenese.

Der für die Hydrolyse und Acidogenese optimale pH-Wert liegt im Bereich von pH 5,2 bis 6,3, der für die Acetogenese und Methanogenese optimale pH-Wert im Bereich von pH 6,7 bis 7,5.

Als zu vergärende Ausgangsstoffe kommen feste und flüssige Substrate zum Einsatz. Sowohl biogene Abfälle aus Industrie, Gewerbe, Landwirtschaft und Haushalten, als auch gezielt zur Erzeugung von Methan angebaute Energiepflanzen werden in Biogasanlagen eingesetzt. Häufig werden in landwirtschaftlichen Biogasanlagen zusätzlich tierische Exkremente dem Prozess zugeführt, um deren Energiepotential zusätzlich auszuschöpfen. Vielfach wird dem Biogasreaktor zu Beginn des Prozesses der Biogaserzeugung Gülle zusammen mit den geernteten Energiepflanzen zugeführt und danach wird der Biogasreaktor ausschließlich mit den geernteten Energiepflanzen gefüttert.

Bislang noch nicht zufriedenstellend funktioniert die Erzeugung von Biogas durch Monovergärung von verschiedenen Früchten oder Rüben oder Knollen oder deren Verarbeitungsrückstände wie z.B. Oliventrester. Oliventrester ist ein Pressrückstand aus der Olivenölproduktion, der bei der Monovergärung als alleiniges Ausgangssubstrat zugeführt wird. Der Presskuchen (Sansa) fällt je nach Herstellungsverfahren in unterschiedlichen Qualitäten an. Die Sansa wird bei der Herstellung von Olivenöl nach dem Prinzip der Kaltpressung produziert.

Die Gewinnung des Öls kann im absatzweisen oder im kontinuierlichen Verfahren erfolgen. Das einstufige Pressen ist die simpelste und am wenigsten mechanisierte Variante. Dagegen ist die kontinuierliche Extraktion die verfahrenstechnisch effektivere Produktionsvariante und unterteilt sich in die Schritte Reinigen, Zerkleinern, Kneten, Pressen und Filtern.

Hierbei werden die Oliven zuerst gereinigt und von Blättern und Ästen befreit. Danach werden die gewaschenen Oliven durch kontinuierlich arbeitende Schneckenpressen oder Hammermühlen zerkleinert, sodass ein breiiges Olivenmus entsteht. Das Mahlen der Oliven richtet sich nach Sorte und Reifegrad. Reife und große Früchte sind in rund 60 Minuten fertig zerkleinert. Kleinere Steinfrüchte müssen 90 Minuten bearbeitet werden. Die Temperatur des zu zerkleinernden Materials darf einen Wert von 28°C nicht überschreiten. Die nachfolgende Bearbeitung findet in einer sogenannten Knetmaschine statt. Die Extraktion bzw. Abtrennung des Öls aus dem Olivenbrei stellt den nächsten Verfahrensschritt dar. Dieser kann entweder durch Pressen, Zentrifugieren oder durch Perkolation (Durchseihen) erfolgen. Das flüssige Zentrifugat bzw. der Presskuchen muss nachfolgend vom Fruchtwasser abgeschieden werden. Dafür wird der sogenannte Most in eine Zentrifuge geleitet, in der sich Wasser und Öl aufgrund ihres unterschiedlichen spezifischen Gewichtes voneinander trennen. Zur Verbesserung des Trennvorgangs wird zusätzlich nur kaltes Wasser in die Zentrifuge gegeben, um einen Qualitätsanspruch zu erhalten. Bis hierher spricht man von einer zweiphasigen Extraktion.

Der letzte Verfahrensschritt bezeichnet die Klärung der Rückstände aus der Zentrifuge. Hier werden die sich sonst absetzenden Reste des Fruchtwassers, die mittels Zentrifuge nicht ausgeschieden werden konnten, abgefiltert (sog. Ölhefe). Anderenfalls würden diese durch ihre Gärung einen unerwünschten, schmutzigen Geschmack verursachen. Das gereinigte Öl aus der dritten Extraktionsstufe wird in Ruhebehältern gelagert, die vor ultravioletten Strahlen schützen. Die Lagerung erfolgt unter Schutzgasatmosphäre, um eine Oxidation zu verhindern.

In der nachfolgenden Tabelle sind in den drei- und zweiphasigen Verfahren die jeweiligen Inhaltsstofffraktionen der Pressrückstände zugeordnet. Daraus ist ersichtlich, dass die dreiphasige Extraktion zu einem geringeren Fettanteil führt.

| Parameter | | Pressen | 3-phasige Extraktion | 2-phasige Extraktion |
|---|---|---|---|---|
| Feuchte | %FM | 27,21 | 50,23 | 56,80 |
| Fette/Öle | %TM | 8,72 | 3,89 | 4,65 |
| Hemicellulose | %TM | 11,0 | 7,92 | 6,63 |
| Lignin | %TM | 14,18 | 10,21 | 8,54 |

Das Potential der Pressrückstände kann anhand der gesamten Anbaufläche grob abgeschätzt werden. In Europa beläuft sich die Anbaufläche auf fast 5.000.000 ha.

| Land | Anbaufläche (ha) |
|---|---|
| Griechenland | 1.065.791 |
| Italien | 1.139.200 |
| Spanien | 2.395.417 |
| Portugal | 365.308 |
| **Total** | **4.965.716** |

Die Entsorgung von Oliventrester ist problematisch. Bei der Lagerung des Oliventresters in Deponien treten Emissionen mit starken Geruchsbelastungen auf.

Bislang verlief die Biogaserzeugung durch Monovergärung von Oliventrester nicht stabil und brach regelmäßig zusammen. In der Literatur wird die Hemmung des Biogasprozesses mit hohen Gehalten an Polyphenolen begründet. In der Gruppe der Polyphenole sind viele Pflanzenstoffe zusammengefasst, zu denen bestimmte Farbstoffe (Flavonoide und Anthocyane und Procyanidine), Benzoesäurederivate, Gerbstoffe (Vanillinsäure, Gallussäure, Protocatechusäure), Zimtsäurederivate (wie Kaffeesäure, p-Cumarsäure), Stilbenderivate (etwa Resveratrol) und Grundbausteine wichtiger Biopolymere (Lignin und Suberin) gehören.

In der Druckschrift Stoyanova et al.: "Difference of the pre-acidification on the anerobic digestion of olive solid waste", ADSW&EC 2011 (International Symposium on Anaerobic Digestion of Solid Waste and Energy Crops, 28.08. - 01.09.2011) in Wien wird auf den inhibitorischen Effekt der Polyphenole auf die Bakterien bei der Biogaserzeugung aus Oliventrester hingewiesen und zur Überwindung ein zweistufiger Fermentationsprozess empfohlen. Zudem wird über die Messung einer niedrigen Armonium-Stickstoff-Konzentration im Fermenter und über das Fehlen einer Pufferkapazität im System berichtet, die einen Abfall des pH-Wertes bei Steigerung der Faulraumbelastung verhindert. Auf die Möglichkeit einer Kofermentation mit einem Substrat mit einem höheren Ammonium-Stickstoffgehalt wird hingewiesen. In diesem Zusammenhang wird auf eine Veröffentlichung betreffend Kofermentation von Abwässern aus der Olivenölproduktion und Schlachthofabfällen Bezug genommen. Die Pufferung des Systems erfordert hohe Stickstoffmengen, die zweckmäßig durch Zugabe von Koferment zugesetzt werden können, zumal das Koferment den Gasertrag steigert.

Ähnliche Probleme treten bei der Erzeugung von Biogas aus anderen Verarbeitungsrückständen von Oliven, Weintrester und anderen Verarbeitungsrückständen von Weintrauben, Verarbeitungsrückständen von Äpfeln, Karotten oder Tomaten, von Rübenschnitzeln oder anderen Verarbeitungsrückständen von Rüben oder von Knollen auf oder sind zu erwarten. Dies gilt auch für die Erzeugung von Biogas aus den genannten Früchten oder Rüben oder Knollen im unverarbeiteten Zustand.

Davon ausgehend liegt der Erfindung die Aufgabe zugrunde, ein verbessertes Verfahren zur Erzeugung von Biogas aus Verarbeitungsrückständen von Früchten oder Rüben oder Knollen oder aus unverarbeiteten Früchten oder Rüben oder Knollen in einem Biogasreaktor zur Verfügung zu stellen.

Die Aufgabe wird durch ein Verfahren mit den Merkmalen von Anspruch 1 gelöst. Vorteilhafte Ausgestaltungen des Verfahrens sind in Unteransprüchen angegeben.

Bei dem erfindungsgemäßen Verfahren zur Erzeugung von Biogas aus Verarbeitungsrückständen von Früchten oder Rüben oder Knollen oder aus unverarbeiteten Früchten oder Rüben oder Knollen als zu vergärendem Ausgangssubstrat in einem Biogasreaktor wird im Falle einer Unterschreitung eines Mindestwertes der Ammonium-Stickstoff-Konzentration im Gärsubstrat ein Prozesshilfsstoff zugeführt, aus dem keine oder keine nennenswerten Biogasmengen gebildet werden und der einen im Gärsubstrat durch Desaminierung Ammonium-Stickstoff freigebenden Stoff umfasst.

Die Erfindung beruht auf der Erkenntnis, dass die ungenügende Stabilität der Biogaserzeugung aus Verarbeitungsrückständen von Früchten oder Rüben oder Knollen oder aus unverarbeiteten Früchten oder Rüben oder Knollen auf einem Mangel an einer für den Stoffwechsel der an der Biogaserzeugung beteiligten Bakterien und anderen Mikroorganismen verfügbaren Stickstoffquelle beruht. Diese Erkenntnis ist insofern überraschend, als in der Literatur die hohen Gehalte an Polyphenol als Ursache für die Störungen der Biogaserzeugung aus Oliventrester angeführt wurden und der analytische Stickstoffgehalt von Oliventrester an sich für die Vergärung ausreichend ist. Zudem gibt es in Biogasanlagen eher Probleme mit zu hohen Ammonium-Stickstoff-Anteilen, die zur Bildung von toxischem Ammoniak führen. Tatsächlich ist jedoch für einen optimalen Biogasprozess die analytische Zusammensetzung aus Kohlenstoff: Stickstoff:Phosphor:Schwefel (C:N:P:S wie 500:15:5:1) nicht ausreichend. Untersuchungen haben vielmehr ergeben, dass eine Verschlechterung des Biogasertrages mit einer Verringerung des Ammonium-Stickstoffes einhergeht, welchen die an der Biogaserzeugung beteiligten Mikroorganismen für den Stoffwechsel benötigen. Gemäß der Erfindung kann durch Zugabe eines unter den im Gärsubstrat im Biogasreaktor herrschenden Bedingungen durch Desaminierung Ammonium-Stickstoff freigebenden Prozesshilfsstoffes zum Gärsubstrat den Mikroorganismen der benötigte Stickstoff für den Stoffwechsel zur Verfügung gestellt werden. Der freigegebene Ammonium-Stickstoff wird von den Mikroorganismen in deren Stoffwechsel verarbeitet. Hierdurch wird die Erzeugung von Biogas wieder stabilisiert. Erfindungsgemäß ist somit die Vergärung von Substraten dennoch möglich, die nach Literaturquellen ausreichende Stickstoffgehalte aufweisen, in der Praxis aber zu Prozessstörungen führen. Der Prozesshilfsstoff ist ein Stoff, der unter den im Gärsubstrat im Biogasreaktor herrschenden Bedingungen Ammonium-Stickstoff freigibt. Alternativ ist der Prozesshilfsstoff eine Stoffzusammensetzung, die als Komponente einen Stoff enthält, der unter den im Gärsubstrat im Biogasreaktor herrschenden Bedingungen Ammonium-Stickstoff freigibt. Der im Gärsubstrat Ammonium-Stickstoff freigebende Stoff wird in dieser Anmeldung auch als "Stickstoffquelle" bezeichnet. Der "Prozesshilfsstoff" ist ein Stoff oder eine Stoffzusammensetzung, aus dem oder der unter den Bedingungen im Biogasreaktor keine oder keine nennenswerten Biogasmengen gebildet werden. Keine nennenswerten Biogasmengen werden aus dem Prozesshilfsstoff gebildet, wenn der darauf entfallende Anteil an dem insgesamt erzeugten Biogas weniger als 2% beträgt.

Die Konzentrationen des Ammonium-Stickstoffes im Gärsubstrat, bei denen die an der Biogaserzeugung beteiligten Mikroorganismen den für ihren Stoffwechsel benötigten Ammonium-Stickstoff nicht mehr erhalten, liegt deutlich unter den Konzentrationen, bei denen gemäß Stoyanova et al. der Ammonium-Stickstoff keine Pufferkapazität bereitstellt. Die von Stoyanova et al. angegebenen Konzentrationen des Ammonium-Stickstoffs sind für den Stoffwechsel der Mikroorganismen hinreichend. Die für den Stoffwechsel der Mikroorganismen benötigten Mengen Ammonium-Stickstoff sind wesentlich geringer als die Mengen, welche für die Bereitstellung einer Pufferkapazität benötigt werden. Die Zugabe verhältnismäßig geringer Mengen des Prozesshilfsstoffs genügt. Eine hinreichende Versorgung der Mikroorganismen mit Ammonium-Stickstoff für den Stoffwechsel ist bereits dann gegeben, wenn nach Zugabe des Prozesshilfsstoffs die Ammonium-Stickstoff-Konzentration in der Biomasse im Biogasreaktor geringfügig ansteigt. Der Anstieg indiziert nämlich, dass eine vollständige Verstoffwechselung des Ammonium-Stickstoffs durch die Mikroorganismen nicht mehr erfolgt. Eine starke Erhöhung der Konzentration des Ammonium-Stickstoffs, wie für eine Pufferung, ist nicht erforderlich.

Bei der Desaminierung wird eine Amminogruppe der Stickstoffquelle als Ammonium oder als Ammmoniak abgespalten. In dieser Anmeldung ist dieser Vorgang allgemein als Freigabe von Ammonium-Stickstoff durch Desaminierung bezeichnet. Ammonium-Stickstoff wird in dieser Anmeldung auch als "freier Stickstoff' oder "verfügbarer Stickstoff" bezeichnet. In der Biogasanalytik misst man Ammonium ($NH_4$) und Ammoniak ($NH_3$) als Summe und drückt den Summenparameter als Ammonium-Stickstoff ($NH_4$-N) aus. Hintergrund ist, dass abhängig vom pH-Wert und von der Temperatur aus $NH_4$ das $NH_3$ und umgekehrt gebildet wird (Gleichgewichtsreaktion).

Der Begriff **Gärsubstrat** wird im Bereich der Erzeugung von Biogas in Biogasanlagen meistens für den in der Vergärung befindlichen Inhalt des Biogasreaktors (auch "Fermenterinhalt" genannt) verwendet. Missverständlich ist, dass auch der Rohstoff zur Biogaserzeugung, das Substrat, sowie das Endprodukt, der Gärrest, gelegentlich als Gärsubstrat bezeichnet werden. In dieser Anmeldung werden die Begriffe "Gärsubstrat" für den in der Vergärung befindlichen Fermenterinhalt, "Substrat" oder "Ausgangssubstrat" oder "Fütterungssubstrat" für den Rohstoff der Biogaserzeugung und "Gärrest" für das Endprodukt der Biogaserzeugung verwendet.

Nach dem Neustart des Verfahrens wird allmählich das Gärsubstrat im Biogasreaktor ausgebildet. Vorzugsweise wird für den Neustart des Verfahrens in den leeren und sauberen Biogasreaktor zunächst eine ausreichende Menge an Fermenterschlamm aus einem laufenden Biogasprozess und oder aus einem Faulprozess der Abwasseraufbereitung und oder eine ausreichende Menge an Rindergülle und oder eine ausreichende Menge an Schweinegülle und eine ausreichende Menge an Wasser eingefüllt. Die flüssige Phase wird auf ca. 30 - 39°C erwärmt, um anschließend langsam das Fütterungssubstrat, d.h. Früchte oder Rüben oder Knollen oder deren Verarbeitungsrückstände, hinzu zu geben. Die Zugabe erfolgt in dieser Startphase mit deutlich geringeren Anteilen an Fütterungssubstrat als für den laufenden Prozess vorgesehen, da sich der Biogasprozess zunächst etablieren und stabilisieren muss. Ein regelmäßiges Rühren sorgt für eine gleichmäßige Verteilung und Homogenisierung der Substrate mit den flüssigen Bestandteilen und die Vermeidung einer Zonenbildung sowie von Temperaturunterschieden im Reaktor. Nur wenn die biologischen Parameter, wie pH, Säuremenge und Gasbildung in Ordnung sind, wird die Fütterungsmenge an Substrat gleichmäßig gesteigert. Da beim Start einer Anlage mit einer Reihe von unterschiedlichen Substanzen, wie Schlämmen und Güllen gearbeitet wird, und der Biogasprozess mit einer geringen Faulraumbelastung gefahren wird, ist in den allermeisten Fällen im Gärsubstrat der erforderliche Gehalt an Ammonium-Stickstoff für eine problemlose Funktion der Mikroorganismen und Bakterien gegeben. Erst bei einer gesteigerten Faulraumbelastung und einem fortgefahrenen Prozess mit der Austragung von Ammoniumstickstoff, tritt die mangelhafte Stickstoffversorgung in Erscheinung. Im Gärsubstrat kommt es zu einem Anstieg des Fos/Tac-Verhältnisses, zu gesteigerten Säuregehalten, zum Abfall des pH-Wertes und zu einem Rückgang der Methangasproduktion.

Um die biologische Verfügbarkeit des Proteingehaltes in pflanzlichen Substraten zu beschreiben lässt sich, da sich die Abbauprozesse im Biogasprozess und im Pansen sehr stark ähneln, zweckmäßigerweise ein bestehendes Bewertungsschema aus der Tierernährung heranziehen. Anhand der Rohproteinfraktionierung wird die Pansen-Verdaulichkeit des Proteins eines pflanzlichen Substrates gut beschrieben und es lässt sich eine allgemein anerkannte Kenngröße für die Verfügbarkeit des Proteingehaltes eines Substrates ermitteln. Bei der chemischen Fraktionierung des Rohproteins von Futtermitteln für Wiederkäuer werden folgende Fraktionen festgestellt (LICITRA et al. 1996)

| Fraktion | Verfügbarkeit | Rohprotein-Fraktion |
|---|---|---|
| A | Im Pansen schnell abbaubar zu (Harnstoff, Peptide, Aminosäuren) Ammoniak | NPN |
| B1 | Im Pansen schnell abbaubar zu Ammoniak | Reinprotein |
| B2 | Im Pansen potenziell vollständig abbaubar | Reinprotein |
| B3 | Im Pansen langsam, nicht unbedingt vollständig abbaubar | Zellwandgebundenes Reinprotein |
| C | Im Pansen und Dünndarm nicht verfügbar | An Lignin, Tannin oder in Maillard Produkten gebundenes Protein |

Das insgesamt vorhandene Rohprotein findet sich in den fünf Fraktionen wieder und wird in der Regel in g/kgTM angegeben. Es ist auch möglich die Summe des gesamten Rohproteins gleich 100% zu setzten und somit die Rohprotein-Fraktionen prozentual darzustellen.

Bei einem Substrat, bei dem die Summe der Rohprotein-Fraktionen B3 und C einen Anteil von mehr als 50% am gesamten Rohprotein hat, kann man von einer schlechten Proteinverfügbarkeit sprechen. Bei einer Monovergärung dieses Substrates muss - aufgrund der schlechten Verfügbarkeit des Rohproteins - mit Problemen in der Biogas-Vergärung gerechnet werden. Je höher der Anteil der Fraktionen B3 und C in einem Substrat ist, umso massiver werden die Probleme und der Mangel an verfügbarem Rohprotein bzw. Stickstoff im Biogasprozess ausfallen.

Die schlechte Verfügbarkeit des Proteins von Oliventrester ist dadurch belegt, dass die Summe der Fraktionen B3 und C über 80% beträgt.

Gemäß einer Ausgestaltung werden dem Biogasreaktor Verarbeitungsrückstände von Oliven insbesondere aus der Produktion von Olivenöl als Ausgangssubstrat zugeführt. Gemäß einer bevorzugten Ausgestaltung wird dem Biogasreaktor Oliventrester als Ausgangssubstrat zugeführt.

Gemäß einer Ausgestaltung werden dem Biogasreaktor als Ausgangssubstrat Weintrester, andere Verarbeitungsrückstände von Weintrauben, Pressrückstände von Äpfeln oder von Karotten oder von Tomaten, andere Verarbeitungsrückstände von Äpfeln oder Karotten oder Tomaten, Rübenschnitzel, andere Verarbeitungsrückstände von Rüben (insbesondere Zuckerrüben, Ertragsrüben, Futterrüben) oder Verarbeitungsrückstände von Knollen (insbesondere Süßkartoffeln) zugeführt. Gemäß einer weiteren Ausgestaltung werden dem Biogasreaktor als Ausgangssubstrat Früchte einer der vorgenannten Arten oder Rüben oder Knollen im unverarbeiteten Zustand zugeführt.

Gemäß einer weiteren Ausgestaltung wird das Biogas in einer Monovergärung erzeugt. Hierbei wird dem Biogasreaktor nur eine einzige Art von Ausgangssubstrat zugeführt, vorzugsweise eines der zuvor genannten Ausgangssubstrate. Die Monovergärung kommt bevorzugt in Regionen zur Anwendung, in denen überwiegend oder ausschließlich spezielle Früchte oder Rüben oder Knollen angebaut bzw. verarbeitet werden. Dies ist insbesondere in den großen Anbaugebieten für Oliven der eingangs erwähnten Mittelmeerländer der Fall. Dort wäre eine Erzeugung von Biogas durch Kofermentation verschiedener Ausgangssubstrate wegen des aufwendigen Transportes regelmäßig unwirtschaftlich.

Gemäß einer anderen Ausgestaltung wird das Biogas in einer Kofermentation erzeugt. Beispielsweise wird eine beliebige Kombination der zuvor genannten Ausgangssubstrate gemeinsam im Biogasreaktor fermentiert.

Gemäß einer weiteren Ausgestaltung beträgt der Mindestwert der Ammonium-Stickstoff-Konzentration im Gärsubstrat ein Wert im Bereich von 50 mg/l bis 400 mg/l. Diese Konzentrationen wurden in der flüssigen Biomasse im Biogasreaktor ermittelt. Dies gilt auch für die nachfolgenden Mindest- und Höchstwerte.

Gemäß einer weiteren Ausgestaltung beträgt der Mindestwert der Ammonium-Stickstoff-Konzentration im Gärsubstrat 50 mg/l, vorzugsweise 100 mg/l.

Gemäß einer weiteren Ausgestaltung wird dem Biogasreaktor Prozesshilfsstoff in einer Menge zugeführt, die so bemessen ist, dass die Ammonium-Stickstoff-Konzentration im Gärsubstrat Höchstwerte von 1.000 mg/l, vorzugsweise 900 mg/l, weiterhin vorzugsweise 750 mg/l, weiterhin vorzugsweise 500 mg/l nicht überschreitet.

Gemäß einer weiteren Ausgestaltung ist die Stickstoffquelle ein durch chemische Verfahren (z.B. durch chemische Synthese) hergestellter Stoff. Gemäß einer anderen Ausgestaltung ist die Strickstoffquelle ein aus natürlichen Vorkommen gewonnener Stoff.

Gemäß einer weiteren Ausgestaltung ist die Stickstoffquelle kein Koferment. Ein Koferment ist ein anaerob abbaubares organisches Substrat, das gemeinsam mit dem Ausgangssubstrat vergärbar ist. Gemäß einer weiteren Ausgestaltung ist die Stickstoffquelle kein Schlachthofabfall.

Ferner wird die Aufgabe durch das nachfolgende Verfahren gelöst.

Bei dem erfindungsgemäßen Verfahren zur Erzeugung von Biogas aus Verarbeitungsrückständen von Früchten oder Rüben oder Knollen oder aus unverarbeiteten Früchten oder Rüben oder Knollen als zu vergärendem Ausgangssubstrat in einem Biogasreaktor wird im Falle einer Unterschreitung eines Mindestwertes der Ammonium-Stickstoff-Konzentration im Gärsubstrat ein Prozesshilfsstoff zugeführt, der kein Koferment ist und der einen im Gärsubstrat durch Desaminierung Ammonium-Stickstoff freigebenden Stoff umfasst.

Gemäß einer weiteren Ausgestaltung enthält der Prozesshilfsstoff als Stickstoffquelle Harnstoff, Ammoniumsalze, Nitratsalze oder eine beliebige Kombination der vorgenannten Stoffe. Harnstoff, Ammoniumsalze und Nitratsalze geben jeweils unter den Bedingungen im Biogasreaktor durch Desaminierung Ammonium-Stickstoff frei. Vorzugsweise sind sie durch chemische Verfahren hergestellt oder aus natürlichen Vorkommen gewonnen.

Gemäß einer weiteren Ausgestaltung ist oder enthält der Prozesshilfsstoff eine Kombination mindestens einer Stickstoffquelle mit einem der Zusatzstoffe Spurenelemente, Fließverbesserer oder einem anderen Betriebshilfsmittel oder einer beliebigen Kombination der genannten Zusatzstoffe. Die Konzentration von Spurenelementen in Oliventrester ist nur gering und kann auch in anderen Ausgangssubstraten gering sein. Durch Zusatz von Spurenelementen kann die Biogaserzeugung aus Oliventrester und anderen Ausgangssubstraten weiter verbessert werden. Fließverbesserer sind vorteilhaft für die Vorbereitung, den Transport und die Dosierung des Prozesshilfsmittels.

Grundsätzlich braucht die Konzentration des Ammonium-Stickstoffs im Gärsubstrat nur einmal ermittelt zu werden, um die Einhaltung des Mindestwerts zu überprüfen und gegebenenfalls den Prozesshilfsstoff zuzugeben. Die Ammonium-Stickstoff-Konzentration innerhalb des Fermenters ist von den jeweils zugeführten Substraten abhängig und kann sich deshalb mit der Fütterung des Fermenters ändern. Ferner kann die biologische Verfügbarkeit des Stickstoffs durch die zugesetzten Substrate und Prozesshilfsstoffe beeinflusst werden und kann sich deshalb mit der Zeit ändern. Zur Erfassung von Änderungen der Konzentrationen des Ammonium-Stickstoffs im Gärsubstrat wird gemäß einer Ausgestaltung des Verfahrens die Konzentration des Ammonium-Stickstoffs im Biogasreaktor in Zeitabständen wiederholt ermittelt. Die jeweils aktuelle Konzentration des Ammonium-Stickstoffs wird mit dem Mindestwert verglichen und einer aktuellen Berechnung der Zugabe zugrunde gelegt.

Die zuzugebende Menge des Prozesshilfsstoffes kann auf verschiedene Weise ermittelt werden. Beispielsweise kann im Falle eines Mangels eine vorgegebene Menge des Prozesshilfsstoffes einmalig oder in Zeitabständen mehrfach zugegeben werden. In einem Zeitabstand kann die Konzentration des Ammonium-Stickstoffs im Biogasreaktor ermittelt werden. Aufgrund der ermittelten Konzentration kann festgestellt werden, ob eine erneute Zugabe der vorgegebenen oder einer abweichenden Menge erforderlich ist. Wird der Mindestwert immer noch unterschritten, kann die vorgegebene Zugabe entsprechend dem Verhältnis des Mindestwerts zur gemessenen aktuellen Konzentration gesteigert werden. Wird der Mindestwert überschritten, kann die vorgegebene Zugabe entsprechend dem Verhältnis des Mindestwertes zur gemessenen aktuellen Konzentration reduziert werden. Auf diese Weise ist eine Optimierung der zuzugebenden Menge möglich. Gemäß einer anderen Ausgestaltung wird nicht anfänglich eine vorgegebene Menge des Prozesshilfsstoffes zugegeben. Vielmehr wird die Menge des zuzugebenden Prozesshilfsstoffs in Abhängigkeit von der Differenz des Mindestwerts und der ermittelten Konzentration ermittelt. Bei einer großen Differenz wird eine entsprechend große Menge des Prozesshilfsstoffs und bei einer geringen Differenz eine entsprechend geringe Menge des Prozesshilfsstoffs in Zeitabständen zugegeben. Zum Ausgleich von Verlusten des Ammonium-Stickstoffs wird gemäß einer weiteren Ausgestaltung die zuzugebende Menge des Prozesshilfsstoffs unter Berücksichtigung der mit den Fermentationsrückständen im Biogasreaktor entnommenen Ammonium-Stickstoffs ermittelt. Hierfür wird gemäß einer Ausgestaltung die Konzentration des Ammonium-Stickstoffs neu ermittelt und die Menge des zuzugebenden Prozesshilfsstoffs neu berechnet.

Gemäß einer Ausgestaltung wird einmalig dem Biogasreaktor eine Menge Prozesshilfsstoff zugeführt, die so bemessen ist, dass eine sofortige Anhebung auf das endgültige Niveau des Ammonium-Stickstoffs erfolgt. In Zeitabständen kann die Gabe wiederholt werden. Sie kann insbesondere nach Ablauf eines Teils der Verweilzeit oder etwa nach Ablauf der Verweilzeit erneut in dem Biogasreaktor gegeben werden.

Gemäß einer weiteren Ausgestaltung wird anfänglich im Biogasreaktor eine Menge Prozesshilfsstoff zugegeben, die geringer ist, als der Bedarf. Die Zugabe wird später dem Bedarf angepasst. Hierdurch kann sich das mikrobiologische System im Biogasreaktor allmählich an die neuen Bedingungen anpassen.

Zugrunde zu legen ist jeweils der Bedarf bezogen auf den Zeitraum, für den die Zugabe erfolgt. Der Zeitabschnitt, in dem eine den Bedarf unterschreitende Menge Prozesshilfsstoff zugegeben wird, ist vorzugsweise geringer als die Verweilzeit des Gärsubstrats im Biogasreaktor, die beispielsweise 1 bis 3 Monate beträgt. Gemäß einer Ausgestaltung wird anfänglich binnen einer bis zwei Wochen nur ein Teil der zuzugebenden Menge Ammonium-Stickstoff zugegeben.

Gemäß einer weiteren Ausgestaltung wird Prozesshilfsstoff in einer gut löslichen Form in den Biogasreaktor eingebracht. Gemäß einer weiteren Ausgestaltung wird der Prozesshilfsstoff gleichmäßig im Biogasreaktor verteilt. Hierdurch kann eine Überschuss- und Mangelsituation in den einzelnen Zonen des Biogasreaktors vermieden werden.

Gemäß einer Ausgestaltung wird der Prozesshilfsstoff kontinuierlich oder einmalig oder wiederholt zugegeben (z.B. in gleichen oder verschiedenen Zeitabständen und/oder in gleichen oder verschiedenen Mengen). Die Mengen werden z.B. durch einmalige oder wiederholte Zugabe eines Depots, das Ammonium-Stickstoff über einen längeren Zeitraum freigibt, zugegeben. Eine einmalige Zugabe von Prozesshilfsstoff kann z.B. erfolgen, um die Biogaserzeugung im Biogasreaktor kurzfristig anzuheben. Langfristig kann dann die Biogaserzeugung durch eine geänderte Fütterung mit Biomasse auf hohem Niveau gehalten werden. Eine kontinuierliche oder wiederholte Zugabe von Prozesshilfsstoff kann z.B. erfolgen, wenn ein Ammonium-Stickstoffmangel der zugefütterten Biomasse langfristig ausgeglichen werden muss.

Die Zugabe des Prozesshilfsstoffs kann in verschiedenen Zeitabständen erfolgen. Gemäß einer Ausgestaltung des Verfahrens erfolgt sie täglich oder in Abständen von mehreren Tagen. Wenn die Zugabe in Zeitabständen erfolgt, die etwa der Verweilzeit (z.B. 1 bis 3 Monate) der Biomasse im Biogasreaktor entsprechen, ist das Ergebnis nicht optimal, weil entweder zu viel Prozesshilfsstoff zugeführt wird oder ein Mangel an Ammonium-Stickstoff eintritt, weil dieser im Wesentlichen verbraucht bzw. dem Fermenter entnommen wird.

Erfolgen die einzelnen Prozessschritte des Biogasprozesses in räumlich getrennten Behältern bzw. Biogasreaktoren, so kann durch die jeweilige Zugabe dem unterschiedlichen Bedarf der in den einzelnen Biogasreaktoren vorliegenden Bakteriengattungen Rechnung getragen werden.

Der Zusatz ist beispielsweise flüssig oder fest, wobei ein fester Zusatz in Form eines Pulvers oder in Form eines Granulates oder mindestens eines anderen Festkörpers zugesetzt werden kann, der schnell oder allmählich im Gärsubstrat zerfällt bzw. sich darin auflöst bzw. Ammonium-Stickstoff freisetzt.

Gemäß einer Ausgestaltung wird der Zusatz speziell in Abhängigkeit von der Menge des zugeführten Ausgangssubstrats und der ermittelten Ammonium-Stickstoff-Konzentration zugegeben. Dem Biogasreaktor wird also eine speziell dem Bedarf angepasste Menge an Zusatz zugegeben, und zwar kontinuierlich, einmalig oder wiederholt.

Nachfolgend wird das Verfahren der Analyse der Stickstoffgehalte im Gärsubstrat näher erläutert:

Probennahme:

Es wird aus dem zu untersuchendem Gärsubstrat eine homogene Probe gezogen, sodass die Zusammensetzung in der Probe identisch ist mit der gesamten Zusammensetzung des Gärsubstrats im Fermenter. Die Probemenge soll insgesamt rund 2kg betragen.

Probenaufbereitung:

Bei jedem Aufarbeitungsschritt der Probe ist für eine ausreichende Durchmischung (Homogenität) zu sorgen.

Bestimmung des Gesamt-Stickstoffgehalts und des Ammonium-Stickstoff-Gehalts: Aus dem Frischmaterial erfolgt die Bestimmung des Gesamt-Stickstoff-Gehalts nach Kjehldahl-Aufschluss mit anschließender Destillation des Ammoniaks und Titration und des Ammonium-Stickstoff-Gehaltes durch direkte Destillation des Ammoniaks und Titration.

Auswertung/Allgemein:

Mit Hilfe des Gehaltes an Gesamtstickstoff und an Ammonium-Stickstoff und der Erkenntnis, welcher Ammonium-Stickstoffgehalt für einen optimalen Biogasprozess nötig ist, kann errechnet werden, ob der Gehalt ausreichend ist oder eine Über- bzw. Unterversorgung vorliegt. Liegt eine Unterversorgung vor, muss dieser durch die Zugabe einer hoch verfügbaren Stickstoffquelle ausgeglichen werden.

Der Mindestwert der Ammoniumkonzentration im Fermenter (Konzentration$[NH_4\text{-}N]_{Mindestwert}$), der für die Berechnung herangezogen wird, ist von verschiedenen Faktoren abhängig und muss im Vorfelde festgelegt werden. Der richtige Mindestwert ist von diversen Faktoren und Umständen im Biogasprozess abhängig, wie Temperatur, Faulraumbelastung, Verweilzeiten, Substratart, Substratbeschaffenheit, Spurenelementversorgung und andere, so dass für die Festlegung des richtigen Mindestwertes bisher Erfahrungen aus der Praxis herangezogen werden.

Formel 1) Berechnung Defizit vor Zugabe des Prozesshilfsstoffs:

$$\text{Konz.}[NH_4\text{-}N]_{\text{Mindestwert}}\ (mg/l)\ -\ \text{Konz.}[NH_4\text{-}N]_{\text{Fermenter}}\ (mg/l) = \text{Start-}$$

$$\text{Defizit}[NH_4\text{-}N]\ (mg/l)$$

Fall 1+2: Ist das Defizit negativ oder null (Defizit $\leq$ 0) ist die $NH_4$-N-Konzentration im Fermenter für einen funktionierenden Biogasprozess ausreichend hoch. Es muss kein Prozesshilfsstoff zugegeben werden.

Fall 3: Ist das Defizit positiv (Defizit > 0) ist die $NH_4$-N-Konzentration im Fermenter für einen funktionierenden Biogasprozess zu niedrig. Es muss Prozesshilfsstoff zugegeben werden.

Formel 2) Berechnung Start-Zugabemenge[$NH_4$-N] Start:

$$\text{Start-Defizit}[NH_4\text{-}N]\ (mg/l)\ \times\ \text{Volumen}[\text{Fermenter}]\ (l)\ /\ 1.000.000\ (mg/kg)$$

$$= \text{Start-Zugabemenge}[NH_4\text{-}N]\ (kg)$$

Die so ermittelte Zugabemenge an $NH_4$-N muss beim Beginn der Behandlung der Anlage in Form eines Prozesshilfsstoffs einmalig zugeführt werden, um den Mindestwert an $NH_4$-N im Fermenter zu erreichen.

Zweckmäßigerweise gibt man die berechnete Menge in mehreren Portionen (2-6) verteilt über 1-2 Wochen hinzu, um im Fermenter keine zu plötzliche Umstellung des biologischen Gesamtsystems zu erhalten.

Nach der Zugabe des Prozesshilfsstoffs muss die durch die Verweilzeit limitierte Aufenthaltsdauer des zugeführten Ammoniums ausgeglichen werden, indem das tägliche ausgetragene Ammonium wieder ergänzt wird.

Formel 3) Berechnung Tages-Zugabemenge[$NH_4$-N] zum Ausgleich der Austragsverluste im laufenden Betrieb:

$$\text{Start-Zugabemenge}[NH_4\text{-}N]\ (kg)\ /\ \text{Verweilzeit}\ (d) = \text{Tages-Zugabemenge}$$

$$[NH_4\text{-}N]\ (kg/d)$$

Durch regelmäßige Messungen des $NH_4$-N-Gehaltes im Fermenter muss kontrolliert werden, ob sich die gewünschte $NH_4$-N-Konzentration im Fermenter eingestellt hat. Vom Prinzip geht man wie beim Start zur erstmaligen Berechnung des Defizits vor, indem man die gefundene Konzentration($NH_4$-N) im Fermenter mit dem angestrebten Mindest-Gehalt vergleicht.

Formel 4) Überprüfung Ammonium-Konzentration im Fermenter nach Zugabe von Prozesshilfsstoff:

$$\text{Konz.}[NH_4\text{-}N]_{\text{Mindestwert}}\ (mg/l)\ -\ \text{Konz.}[NH_4\text{-}N]_{\text{Fermenter}}\ (mg/l)\ =$$

$$\text{Defizit}[NH_4\text{-}N]\ (mg/l)$$

Fall 1: Ist das Defizit negativ (Defizit < 0) ist die $NH_4$-N-Konzentration im Fermenter für einen funktionierenden Biogasprozess ausreichend hoch, aber es wird ein Überschuss an $NH_4$-N hinzu gegeben. Die tägliche Zugabemenge an $NH_4$-N sollte entsprechend reduziert werden.

Fall 2: Ist das Defizit null oder nahe bei null (Defizit = 0) ist die $NH_4$-N-Konzentration im Fermenter für einen funktionierenden Biogasprozess genau richtig eingestellt. Die tägliche Zugabemenge sollte konstant gehalten werden.

<u>Fall 3:</u> Ist das Defizit positiv (Defizit > 0) ist die $NH_4$-N-Konzentration im Fermenter für einen funktionierenden Biogasprozess zu niedrig. Es muss täglich mehr Prozesshilfsstoff als bisher zugegeben werden.

Formel 5) Berechnung tägliche Ergänzungs-Zugabemenge[$NH_4$-N]:

$$\textbf{Defizit[NH}_4\textbf{-N] (mg/l)} \ \ \textbf{x} \ \ \textbf{Volumen[Fermenter] (l)} \ / \ \textbf{1.000.000 (mg/kg)} /$$

$$\textbf{Verweilzeit = Ergänzungs-Zugabemenge[NH}_4\textbf{-N] (kg/d)}$$

Die so ermittelte tägliche Ergänzungs-Zugabemenge[$NH_4$-N] muss zusätzlich zur zuvor berechneten Tages-Zugabemenge[$NH_4$-N] (Formel 4) dem Fermenter gefüttert werden. Es ergibt sich als Summe die angepasste Tages-Zugabemenge[$NH_4$-N], die von nun an täglich gefüttert werden sollte.

Formel 6) Berechnung angepassten täglichen Ergänzungs-Zugabemenge[$NH_4$-N]:

$$\textbf{Tages-Zugabemenge[NH}_4\textbf{-N] (kg/d)} \ + \ \textbf{Ergänzungs-Zugabemenge[NH}_4\textbf{-N]}$$

$$\textbf{(kg/d) = angepasste Tages-Zugabemenge[NH}_4\textbf{-N] (kg/d)}$$

In regelmäßigen Abständen, die sich danach richten, wie gut man die gewünschte Konzentration an $NH_4$-N im Fermenter findet (1-4 Wochen), sollte die $NH_4$-N-Konzentration im Fermenter gemessen werden. Mit Hilfe der Analysenergebnisse sollte eine <u>Überprüfun der Ammonium-Konzentration im Fermenter nach Zugabe von Prozesshilfsstoff,</u> wie vorne beschrieben, erfolgen und entsprechende Maßnahmen ergriffen werden.

Da die verschiedenen Prozesshilfsstoffe unterschiedliche Mengen an $NH_4$-N freisetzen können müssen die gewünschten Zugabemengen $NH_4$-N auf den Prozesshilfsstoff umgerechnet werden. Dafür benötigt man die Konzentration an $NH_4$-N im Prozesshilfsstoff in %

Formel 7) Umrechnung Zugabemenge[$NH_4$-N] in Zugabemenge[Prozesshilfsstoff]:

$$\textbf{Zugabemenge[NH}_4\textbf{-N] (kg)} \ \ \textbf{x} \ \ \textbf{100\%} \ / \ \textbf{NH}_4\textbf{-N[Prozesshilfsstoff] (\%) =}$$

$$\textbf{Zugabemenge[Prozesshilfsstoff] (kg)}$$

<u>Praktisches Beispiel:</u>

Ein Fermenter mit Oliventrester als Monovergärung und 2500m$^3$ Volumen und 90 Tagen Verweilzeit weist einen $NH_4$-N Wert von 0,04g/l bzw. 40mg/l auf. Der Mindestgehalt an $NH_4$-N wird mit 250mg/l festgelegt.

$$\textbf{Formel 1)} \quad \textbf{250mg/l} \ - \ \textbf{40mg/l} = \underline{\textbf{210mg/l}} = \textbf{Start-Defizit[NH}_4\textbf{-N]} > \textbf{0} \ \ \text{➜ Zugabe}$$

$$\text{notwendig}$$

$$\textbf{Formel 2)} \quad \textbf{210mg/l} \quad \textbf{x} \quad \textbf{2.500.000l} \ / \ \textbf{1.000.000mg/kg} = \underline{\textbf{525kg}} = \textbf{Start-Zugabe-}$$

$$\textbf{menge[NH}_4\textbf{-N] (kg)}$$

Formel 3)    525kg / 90d = 5,83kg/d = **Tages-Zugabemenge[NH₄-N] (kg/d)**

Zwei Wochen nach den Zugaben wird ein $NH_4$-N-Wert von 190mg/l ermittelt:

Formel 4)    250mg/l - 190mg/l = 60mg/l = **Defizit[NH₄-N]**

Formel 5)    60mg/l x 2.500.0001 / 1.000.000mg/kg / 90d = 1,66kg/d =

**Ergänzungs-Zugabemenge[NH₄-N] (kg/d)**

Formel 6)    5,83kg/d + 1,66kg/d = 7,49kg/d = **angep. Tages-Zugabemenge**

**[NH₄-N] (kg/d)**

Die Zugabe des $NH_4$-N soll mit reinem Harnstoff ($NH_4$-N-Gehalt = 46,66%) erfolgen:

Formel 7)    7,49kg/d x 100% / 46,66% = 16,05kg/d = **Zugabemenge[Prozess-**

**hilfsstoff] (kg)**

Die berechneten täglichen Zugabemengen an $NH_4$-N können auch in anderen Abständen z.B. alle drei Tage zugegeben werden, dafür sind die täglichen Mengen entsprechend zusammen zu fassen.

Ergebnisse von Laboruntersuchungen zur Vergärung von Olivenpresskuchen

**Beispiel 1:**

Zwei Laborfermenter wurden mit Olivenpresskuchen aus dem Extraktionsverfahren als Monosubstrat gefüttert. Im Rahmen des Versuches wurde die Fütterung mit einer beginnenden Raumlast von 3 kg organischer Trockenmasse je Kubikmeter und Tag wöchentlich um 0,5 Einheiten gesteigert. Bis zu einer Raumlast von 8,0 kg oTM/(m³*d) ist die Prozessführung unauffällig.

Die Raumlast von 8,5 kg oTM/(m³*d) wurde aufgrund eines starken Anstieges des FOS/TAC-Wertes nach kurzer Zeit wieder auf 8,0 kg oTM/(m³*d) zurückgenommen. Aber erst ein Aussetzen der Fütterung führte zu einer signifikanten Senkung des FOS/TAC-Wertes. Die Wiederaufnahme der Fütterung auf halbem Niveau der höchsten stabilen Dosierung führte zu sofortigen Prozessstörungen in Form eines steigenden FOS/TAC-Wertes. Die Messung des Ammonium-Stickstoff-Gehaltes ergab eine Wert von 0,24g/l und lag somit deutlich unter dem Ammoniumstickstoffgehalt von 1,2 - 0,8g/l zu früheren Zeitpunkten des Versuches. Die Zugabe des Prozesshilfsstoffes senkte den FOS/TAC auf das Niveau vor der Prozessstörung und die Fütterung konnte ohne erkennbare Probleme gesteigert werden. Die Gasbildung stabilisierte sich ab der 25. Woche auf dem Niveau vor Prozessstörung.

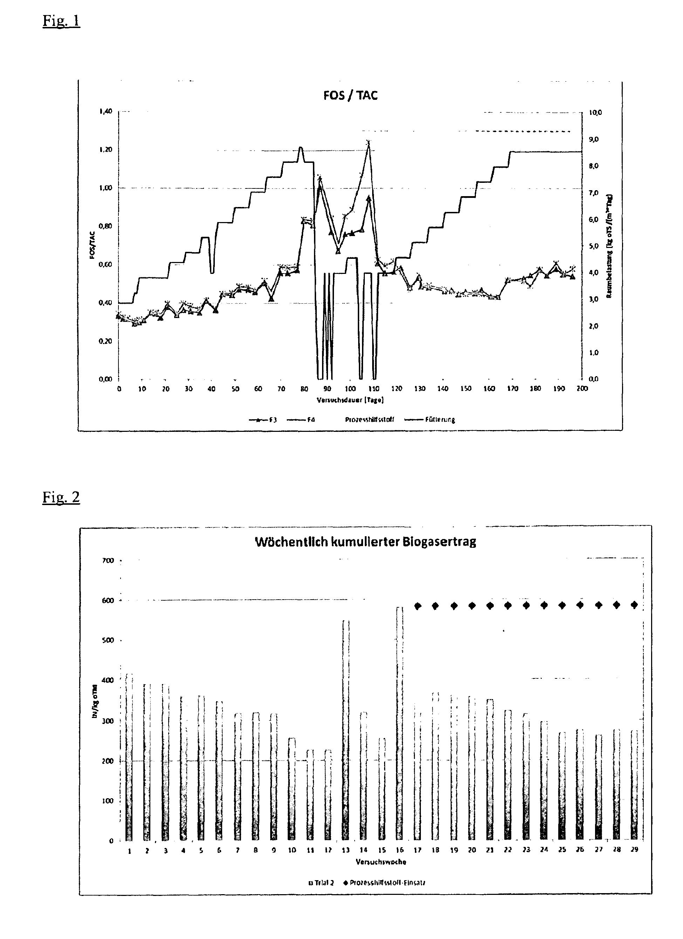

Die Entwicklung der FOS/TAC-Werte (Fig. 1) und der Gasentwicklung (Fig. 2) vor und nach Einsatz des Prozesshilfsstoffs sind in Diagrammen dargestellt. Darin sind der Verlauf der FOS/TAC-Werte über der Zeit sowie die Raumbelastung im Hauptfermenter dargestellt.

Die jeweiligen Messwerte sind durch Kurven verbunden. Es ist gut erkennbar, dass der FOS/TAC in der Biogasanlage sich innerhalb von zwei Tagen nach der Prozesshilfsstoffzugabe normalisiert.

Zu den Fos/Tac-Werten ist folgendes anzumerken:

Der FOS/TAC hat sich bei der Analyse von Biogasfermentern bewährt und wird bei praktisch allen Untersuchungen durchgeführt. Zum Teil erfolgen diese Analysen auch schon auf den Biogasanlagen durch die Installation einfacher Messgeräte. Der Quotient beschreibt das Verhältnis der flüchtigen organischen Säuren zur alkalischen Pufferkapazität als Maß für die Versäuerungsgefahr von Biogasanlagen. Die FOS/TAC-Gehalte ermittelt man durch Titration mit einer bestimmten Säure. So werden 20ml zentrifugierter Fermenterprobe mit ca. 80ml Wasser verdünnt. Im Anschluss wird unter Rühren und unter ständiger pH-Wert-Messung mit 0,1n Schwefelsäure titriert. Man notiert den Verbrauch an Schwefelsäure (ml 0,1n Schwefelsäure) bis zum pH-Wert von 5,0 und titriert weiter bis zum pH-Wert von 4.4. Man notiert den Verbrauch an Schwefelsäure (ml 0,1n Schwefelsäure) von pH 5,0 bis pH 4.4. Die notierten Werte zur Einstellung werden in die folgende empirische Formel eingegeben und berechnen so die gesuchten Fos/Tac-Werte:

$$TAC = H_2SO_4\text{-Verbrauch vom Beginn bis pH 5 in ml x 250}$$

$$FOS = ((H_2SO_4\text{-Verbrauch von pH5 bis pH 4,4 in ml x 1,66}) - 0,15) \times 500.$$

Der Quotient aus FOS/TAC sollte unter 0,3 liegen, was bedeutet, dass die Gewichtung zwischen Säure und Puffer ausgewogen ist. Steigt der Wert über 0,4, sind zu viele Säuren für den aktuellen Carbonatpuffer vorhanden. Dies ist ein eindeutiges bekanntes Anzeichen für einen nicht optimalen Biogasprozess, häufig dadurch ausgelöst, dass die Säuren nicht schnell genug oder nicht ausreichend genug abgebaut werden.

**Beispiel 2:**

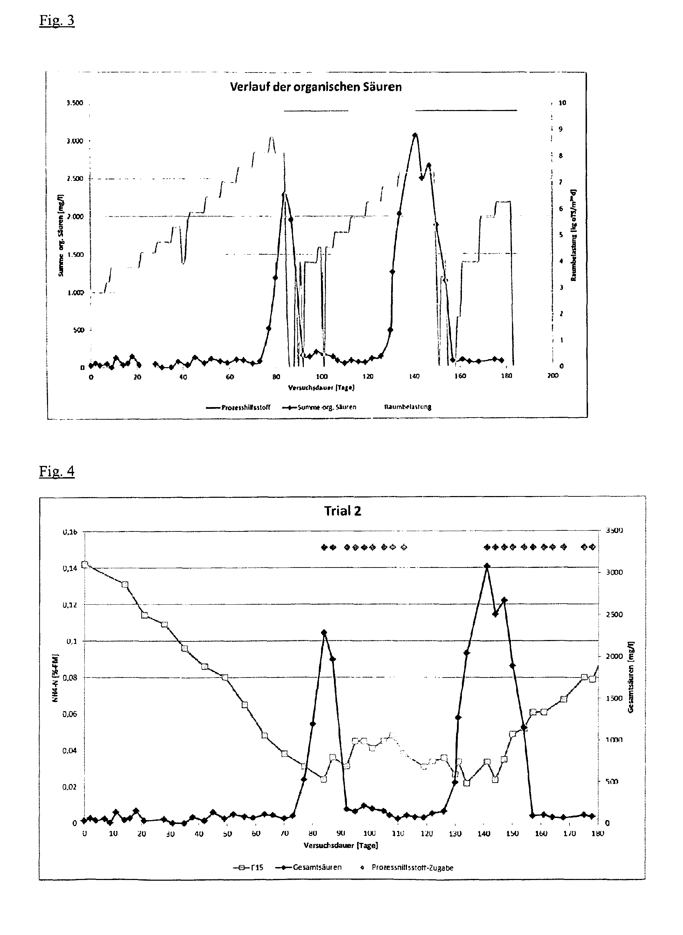

Ein Laborfermenter wurde mit Olivenpresskuchen als Monosubstrat gefüttert. Im Rahmen des Versuchs wurde die Fütterung mit einer beginnenden Raumlast von 3 kg organischer Trockenmasse je Kubikmeter und Tag wöchentlich um 0,5 Einheiten gesteigert. Bei einer Raumlast von 8 kg oTM/m3d sind die Gesamtsäuren von einem unauffälligen Level < 100 mg/L innerhalb kürzester Zeit auf > 2.000 mg/L angestiegen (Fig. 3 und 4).
Die Reduktion der Fütterungsmenge zeigte keinen Einfluss auf den Säuregehalt.
Der Ammoniumstickstoffgehalt lag zu diesem Zeitpunkt des Versuches bei 0,2g/l und war in den vorangegangenen Wochen stetig abgefallen.
Der Einsatz des Prozesshilfsmittels führte hingegen zu einem sofortigen Abbau der Säuren und einen moderaten Anstieg des Ammoniumstickstoffgehaltes auf über 0,4g/l. Eine Wiederaufnahme der Fütterung erfolgte mit 4 kg oTM je $m^3$ und Tag mit einer Steigerung von 0,5 kg oTM je $m^3$ und Tag. Nach Absetzen des Prozesshilfsstoffes zeigten sich ca. 14 Tage später wieder Prozessstörungen in Form von ansteigenden Säuren. Ein Einsatz des Prozesshilfsstoffes konnte auch diese Störung wieder beseitigen.
Unterer Grenzwert, oberer Grenzwert und Sollbereich der Ammoniumstickstoff-Konzentration im Biogasreaktor sind in der nachstehenden Tabelle zusammengefasst:

| Verbindung | unterer Grenzwert | Sollbereich | oberer Grenzwert |
|---|---|---|---|
| | mg/L | mg/l | mg/l |
| Ammonium-Stickstoff | 50 | 150-500 | 1000 |

**Patentansprüche**

1. Verfahren zur Erzeugung von Biogas aus Verarbeitungsrückständen von Früchten oder Rüben oder Knollen oder aus unverarbeiteten Früchten oder Rüben oder Knollen als zu vergärendes Ausgangssubstrat in einem Biogasreaktor, bei dem im Falle einer Unterschreitung eines Mindestwertes der Ammonium-Stickstoff-Konzentration im Gärsubstrat ein Prozesshilfsstoff zugeführt wird, aus dem keine oder keine nennenswerten Biogasmengen gebildet werden und der einen im Gärsubstrat durch Desaminierung Ammonium-Stickstoff freigebenden Stoff umfasst.

2. Verfahren nach Anspruch 1, bei dem ein zu vergärendes Ausgangssubstrat mit einem Anteil der Summe der Rohprotein-Fraktionen B3 und C an sämtlichen Rohprotein-Fraktionen von mehr als 50% dem Biogasreaktor zugeführt wird.

3. Verfahren nach Anspruch 1 oder 2, bei dem Oliventrester oder andere Verarbeitungsrückstände von Oliven oder unverarbeitete Oliven als zu vergärendes Ausgangssubstrat dem Biogasreaktor zugeführt werden.

4. Verfahren nach einem der Ansprüche 1 bis 3, bei dem Weintrester oder andere Verarbeitungsrückstände von Weintrauben, Pressrückstände oder andere Verarbeitungsrückstände von Äpfeln oder Tomaten oder Karotten, Rübenschnitzel oder andere Verarbeitungsrückstände von Rüben, Verarbeitungsrückstände von Knollen oder die genannten Früchte oder Rüben oder Knollen im unverarbeitetem Zustand als zu vergärendes Ausgangssubstrat dem Biogasreaktor zugeführt wird.

**5.** Verfahren nach einem der Ansprüche 1 bis 4, bei dem das Biogas in einer Monovergärung erzeugt wird.

**6.** Verfahren nach einem der Ansprüche 1 bis 5, bei dem der Mindestwert der Ammonium-Stickstoff-Konzentration im Gärsubstrat einen Wert im Bereich von 50 mg/l bis 400 mg/l ist.

**7.** Verfahren nach Anspruch 6, bei dem der Mindestwert der Ammonium-Stickstoff-Konzentration im Gärsubstrat 50 mg/l, vorzugsweise 100 mg/l beträgt.

**8.** Verfahren nach einem der Ansprüche 1 bis 7, bei dem Gärsubstrat Prozesshilfsstoff in einer Menge zugeführt wird, die so bemessen ist, dass die Ammonium-Stickstoff-Konzentration im Gärsubstrat 1.000 mg/l, vorzugsweise 750 mg/l, weiterhin vorzugsweise 500 mg/l nicht überschreitet.

**9.** Verfahren nach einem der Ansprüche 1 bis 8, bei dem dem Gärsubstrat Prozesshilfsstoff in einer Menge zugeführt wird, die so bemessen ist, dass die Ammonium-Stickstoff-Konzentration im Gärsubstrat im Biogasreaktor im Bereich von 150 bis 500 mg/l angesiedelt ist.

**10.** Verfahren nach einem der Ansprüche 1 bis 9, bei dem der durch Desaminierung Ammonium-Stickstoff freigebende Stoff ein durch chemische Verfahren hergestellter Stoff oder ein aus natürlichen Vorkommen gewonnener Stoff ist.

**11.** Verfahren nach einem der Ansprüche 1 bis 10, dem der Prozesshilfsstoff Harnstoff, Ammoniumsalze, oder Nitratsalze als Stickstoffquelle oder eine beliebige Kombination der vorgenannten Stickstoffquellen enthält.

**12.** Verfahren nach einem der Ansprüche 1 bis 11, dem der Prozesshilfsstoff mindestens eine Stickstoffquelle in Kombination mit einem der Zusatzstoffe Spurenelemente, Fließverbesserer oder einem anderen Betriebshilfsmittel oder einer beliebigen Kombination der genannten Zusatzstoffe enthält.

Fig. 1

Fig. 2

Fig. 3

Verlauf der organischen Säuren

Fig. 4

Trial 2

Europäisches
Patentamt

European
Patent Office

Office européen
des brevets

**EUROPÄISCHER RECHERCHENBERICHT**

Nummer der Anmeldung

EP 12 00 5002

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 101 153 289 A (UNIV SHANGHAI JIAOTONG [CN]) 2. April 2008 (2008-04-02) * das ganze Dokument * ----- | 1-12 | INV. C12P5/02 |
| X | CN 101 509 016 B (UNIV SOUTHWEST [CN] UNIV SOUTHWEST) 28. März 2012 (2012-03-28) * das ganze Dokument * ----- | 1-12 | |
| X | CN 101 575 618 A (AGRO MACHINERY RES INST OF CHI [CN]) 11. November 2009 (2009-11-11) * das ganze Dokument * ----- | 1-12 | |
| X | CN 101 899 473 B (UNIV CHONGQING) 25. Januar 2012 (2012-01-25) * das ganze Dokument * ----- | 1-12 | |
| A | WO 2008/090231 A2 (SACHTLEBEN CHEMIE GMBH [DE]; VOLLMUTH STEFAN [DE]; SCHOETTLER ARND [DE] 31. Juli 2008 (2008-07-31) * Ansprüche 1-16 * ----- | 1-12 | RECHERCHIERTE SACHGEBIETE (IPC) |
| A | WO 2008/038860 A1 (UNIV KOREA IND & ACAD COOP [KR]; CHOI EUI-SO [KR]) 3. April 2008 (2008-04-03) * Ansprüche 1-9 * ----- | 1-12 | C12P |

Der vorliegende Recherchenbericht wurde für alle Patentansprüche erstellt

| Recherchenort | Abschlußdatum der Recherche | Prüfer |
|---|---|---|
| Berlin | 15. November 2012 | Siatou, Evangelia |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 12 00 5002

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-11-2012

| Im Recherchenbericht angeführtes Patentdokument | Datum der Veröffentlichung | Mitglied(er) der Patentfamilie | Datum der Veröffentlichung |
|---|---|---|---|
| CN 101153289    A | 02-04-2008 | KEINE | |
| CN 101509016    B | 28-03-2012 | KEINE | |
| CN 101575618    A | 11-11-2009 | KEINE | |
| CN 101899473    B | 25-01-2012 | KEINE | |
| WO 2008090231   A2 | 31-07-2008 | KEINE | |
| WO 2008038860   A1 | 03-04-2008 | KR   20080028247 A<br>WO   2008038860 A1 | 31-03-2008<br>03-04-2008 |

EPO FORM P0461

Für nähere Einzelheiten zu diesem Anhang : siehe Amtsblatt des Europäischen Patentamts, Nr.12/82

# EP 2 682 470 A1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **MAURER, M. ; WINKLER, J.-B.** Biogas. Theoretische Grundlagen, Bau und Betrieb von Anlagen. Springer-Verlag, 1982
- **SAHM.** Biologie der Methanbildung. *Chem.-Ing. Tec.,* 1981, vol. 53 (11), 854-863
- **DRUCKSCHRIFT STOYANOVA et al.** Difference of the pre-acidification on the anerobic digestion of olive solid waste. *ADSW&EC 2011 (International Symposium on Anaerobic Digestion of Solid Waste and Energy Crops,* 28. August 2011